# DEMANDE DE BREVET EUROPEEN

(11) **EP 4 767 949 A1**
(43) Date de publication de la demande: **01.07.2026**
(21) Numéro de dépôt: 25221234.5
(22) Date de dépôt: 05.12.2025
(51) Int. Cl.: A61B 8/00, A61B 8/08, A61B 5/00, A61B 5/02

(54) **DISPOSITIF COMPRENANT UN TRANSDUCTEUR ULTRASONORE**

(30) Priorité: 27.12.2024 FR 2415315
(71) Demandeur: Withings, 92130 Issy-les-Moulineaux (FR)
(72) Inventeur: Criton, Aline, 92130 Issy-les-Moulineaux (FR); Menanteau, Matthieu, 92130 Issy-les-Moulineaux (FR)
(74) Mandataire: Withings IP

(57) **Abrégé**

Il est proposé un dispositif configuré pour être positionné sur un membre (M) d'un utilisateur, le dispositif (100) comprenant :
• un capteur physiologique (150) configuré pour générer un signal cardiaque caractéristique d'une activité cardiaque de l'utilisateur,
• un transducteur ultrasonore (180) configuré pour générer un signal Doppler caractéristique d'une circulation sanguine dans le membre (M),
• une unité de contrôle (140) configurée pour :
∘ recevoir un signal cardiaque du capteur physiologique (150),
∘ analyser le signal cardiaque reçu, et
∘ en réponse à une détection d'une suspicion d'arythmie cardiaque dans l'analyse du signal cardiaque, activer le transducteur ultrasonore (180) pour générer un signal Doppler.

## Description

### Domaine technique

La présente invention concerne le domaine des dispositifs de mesure comprenant un transducteur ultrasonore, notamment les dispositifs portables (« *wearable »* en anglais).

### Technique antérieure

L'accident vasculaire cérébral (ou « AVC ») cryptogénique est un infarctus cérébral dont la cause reste inconnue après une évaluation médicale approfondie. La stratégie immédiate devant un AVC aigu doit être fondée sur la recherche d'une indication de revascularisation cérébrale médicamenteuse et/ou mécanique. Après cette phase aiguë, un bilan étiologique exhaustif doit être réalisé pour identifier si possible la cause de l'AVC et proposer une prévention secondaire adaptée. Avec cette définition assez large, on estime que 25 % des AVC ischémiques (type d'accident vasculaire cérébral qui survient lorsque le flux sanguin vers une partie du cerveau est bloqué, généralement par un caillot) peuvent être classés comme cryptogéniques *(*Hart RG, Diener HC, Coutts SB, Easton JD, Granger CB, O'Donnell MJ, et al. Embolic strokes of undetermined source: the case for a new clinical construct. Lancet Neurol 2014;13:429-38*).* Les données épidémiologiques nord-américaines indiquent qu'environ 20 % des AVC sont des récidives d'AVC *(*Virani SS, Alonso A, Aparicio HJ, Benjamin EJ, Bittencourt MS, Callaway CW, et al.; American Heart Association Council on Epidemiology and Prevention Statistics Committee and Stroke Statistics Subcommittee. Heart Disease and Stroke Statistics-2021 Update: A Report From the American Heart Association. Circulation. 2021;143:e254-e743)*.*

La fibrillation auriculaire (ou « FA ») est une cause majeure connue d'AVC cardio-embolique. Des études ont montré que les passages en FA transitoire étaient fréquents dans une population à risque élevé d'AVC ou ayant eu un accident ischémique transitoire ou un AVC, mais sans antécédent de FA documentée *(*Daoud EG, Glotzer TV, Wyse DG, Ezekowitz MD, Hilker C, Koehler J, et al. Temporal relationship of atrial tachyarrhythmias, cerebrovascular events, and systemic emboli based on stored device data: a subgroup analysis of TRENDS. Heart Rhythm 2011;8:1416-23 *,* Brambatti M, Connolly SJ, Gold MR, Morillo CA, Capucci A, Muto C, et al. Temporal relationship between subclinical atrial fibrillation and embolie events. Circulation 2014;129:2094-9*).* Cette FA infra-clinique augmente de 2.5 fois le risque d'AVC ischémique ou d'embolie systémique. La FA peut être responsable de près d'un tiers des AVC ischémiques dans certaines populations *(*Ntaios G, Papavasileiou V, Milionis H, Makaritsis K, Vemmou A, Koroboki E, et al. Embolic Strokes of Undetermined Source in the Athens Stroke Registry: An Outcome Analysis. Stroke. 2015; 46: 2087-93*).*

Si la surveillance rythmique hospitalière n'apporte pas de réponse diagnostique, un holter ECG (appareil portable qui enregistre l'activité électrique du cœur en continu sur une période prolongée, généralement 24 à 48 heures) est recommandé. Les enregistrements ECG réalisés après l'hospitalisation dépassent rarement 48 heures pour des raisons logistiques. En effet, très peu d'appareils holters ECG de longue durée sont disponibles.

Une méta-analyse d'une trentaine d'études prospectives a montré que la prévalence de la FA était de 5% avec les enregistrements inférieurs à 72 heures contre 15% avec les enregistrements supérieurs à 7 jours *(*Dussault C, Toeg H, Nataan M, Wang ZJ, Roux JF, Secemsky E. Electrocardiographic monitoring for detecting atrial fibrillation after ischemic stroke or transient ischemic attack: systematic review and meta-analysis. Circ Arrhythm Electrophysio. 2016;8:263-9*.).* L'usage d'un moniteur cardiaque implantable (MCI) permet de passer à un taux de détection de FA de 30% sur trois ans comparé à un taux de 3% dans le groupe contrôle (Sanna T, Diener HC, Passman RS, Di Lazzaro V, Bernstein RA, Morillo CA, et al. Cryptogenic stroke and underlying atrial fibrillation. N Engl J Med 2014;26:370:2478-86*.).* Une indication d'implantation de MCI est retenue en France pour le diagnostic étiologique des AVC ischémiques, sans qu'une source cardio-embolique ou un trouble de la coagulation n'ait pu être mis en évidence.

Ces accidents vasculaires cérébraux ischémiques sont le plus souvent dus à aux micro-embolies générées pendant les épisodes de fibrillation chez ses patients souffrant de FA. Ces micro-emboles peuvent être détectées à l'aide d'une échographie Doppler transcranienne. Dans cette technique, un signal ultrasonore pulsé à fréquence constante balaye un volume de sang dans l'artère cérébrale moyenne, et les micro-emboles se déplaçant dans son volume d'échantillonnage produisent une réflexion transitoire décalée par rapport à l'effet Doppler avec une amplitude très élevée appelée HITS pour « *high intensity transient signal »* en anglais (ou « signal transitoire de haute intensité » en français).

Les échographies Doppler transcrâniennes ont plusieurs contraintes matérielles et organisationnelles. En particulier, afin de réaliser ces données d'imagerie Doppler, il faut avoir accès à des échographes qui ne sont pas des dispositifs que l'on peut intégrer facilement dans le quotidien d'un patient permettent de surveiller facilement sur le long terme les patients à risque de FA. La réalisation de ce type d'examen d'imagerie ne peut se faire que par un médecin radiologue ou un échographiste spécialisé en imagerie Doppler transcrânienne. Ces examens ne peuvent pas être fait de façon continue ou lorsqu'un épisode de FA est présent d'une part parce que les épisodes de FA sont souvent asymptomatiques et d'autre part parce qu'il est difficile d'un point de vue organisationnel d'avoir accès à des examens d'imagerie cérébrale au moment de l'épisode de FA même si l'épisode est détecté.

On connait également quelques tentatives d'incorporer un capteur ultrason dans une montre, comme décrit notamment dans le document US20240130617A1. Toutefois ces montres présentent de nombreuses limitations techniques rendant leur utilisation concrète par un patient difficile.

### Exposé de l'invention

La présente description vise donc à proposer un dispositif permettant de détecter la présence de ces micro-embolies lors d'épisodes d'arythmie plus facilement et plus systématiquement, afin d'évaluer et de prévenir les risques dus à un accident vasculaire cérébral.

A cet effet, la présente description concerne un dispositif configuré pour être positionné sur un membre d'un utilisateur, le dispositif comprenant : un capteur physiologique configuré pour générer un signal cardiaque caractéristique d'une activité cardiaque de l'utilisateur, un transducteur ultrasonore configuré pour générer un signal Doppler caractéristique d'une circulation sanguine dans le membre, une unité de contrôle configurée pour : recevoir un signal cardiaque du capteur physiologique, analyser le signal cardiaque reçu, et en réponse à une détection d'une suspicion d'arythmie cardiaque dans l'analyse du signal cardiaque, activer le transducteur ultrasonore pour générer un signal Doppler.

Le dispositif selon la présente description permet une détection non invasive et précise des micro-emboles chez des utilisateurs atteints de fibrillation auriculaire. En intégrant un transducteur ultrasonore dans un dispositif, tel que par exemple une montre connectée, un brassard de tensiomètre ou une balance, il est possible de détecter la présence et la quantité de micro-emboles dans le sang à la suite de la détection d'une arythmie, ce qui offre une opportunité de prévenir les AVC chez les utilisateurs souffrant de FA. En particulier, la détection d'une micro-embole peut déclencher une alerte, permettent une intervention médicale rapide, ce qui est crucial pour prévenir les dommages causés par les AVC.

En particulier, l'activation du transducteur ultrasonore temporaire (typiquement quelques minutes) et l'acquisition d'un signal Doppler uniquement en cas de détection d'arythmie permet une utilisation de la batterie du dispositif optimisée rendant possible un suivi long-terme et continu pour l'utilisateur.

Enfin, le dispositif étant non invasif et pouvant être portable, il permet en outre d'améliorer le confort et la qualité de vie de l'utilisateur, ce qui augmente l'adhésion de l'utilisateur à l'utilisation régulière du dispositif.

Dans un mode de réalisation, une suspicion d'arythmie cardiaque est une probabilité de présence d'une arythmie supérieure à un seuil prédéterminée, par exemple 50% de chance.

Dans un mode de réalisation, le transducteur ultrasonore est une sonde Doppler.

Dans un mode de réalisation, l'arythmie est une fibrillation auriculaire.

Dans un mode de réalisation, le capteur optique est configuré pour effectuer une mesure du signal cardiaque en continu et l'unité de contrôle est configurée pour analyser le signal cardiaque en continu.

Dans un mode de réalisation, l'analyse du signal cardiaque se fait à l'aide d'une fenêtre temporelle glissante.

Dans un mode de réalisation, le capteur physiologique est un capteur optique.

Dans un mode de réalisation, le capteur physiologique et/ou un capteur ECG et/ou un capteur oscillométrique, un ballistographe ou ballistocardiographe, BCG, et/ou un capteur impédancemétrique, IPG.

Dans un mode de réalisation, le dispositif est un dispositif portable.

Dans un mode de réalisation, le capteur optique comprend au moins une source de lumière et au moins un récepteur de lumière

Dans un mode de réalisation, le signal cardiaque est un signal photo-pléthysmographique.

Dans un mode de réalisation, le capteur optique est configuré pour effectuer une mesure du signal cardiaque de manière périodique, notamment à une fréquence comprise entre une mesure toutes les minutes et une mesure toutes les 3 minutes, ou 5 minutes, ou 10 minutes.

Dans un mode de réalisation, le signal cardiaque est un signal oscillométrique.

Dans un mode de réalisation, le signal cardiaque est un signal BCG.

Dans un mode de réalisation, le signal cardiaque est un signal IPG.

Dans un mode de réalisation, le capteur oscillométrique est activé lors d'une mesure de tension artérielle.

Dans un mode de réalisation, le capteur ECG comprend au moins deux électrodes ECG en matériau conducteur.

Dans un mode de réalisation, l'unité de contrôle est électriquement connecté aux deux électrodes ECG, et configuré pour effectuer un électrocardiogramme et détecter une arythmie dans l'électrocardiogramme effectué.

Dans un mode de réalisation, le module ECG met en œuvre un algorithme d'apprentissage profond.

Dans un mode de réalisation, le signal Doppler est généré concomitamment au signal cardiaque ou moins de 10 secondes, voire 5 secondes après le signal cardiaque.

Dans un mode de réalisation, le transducteur ultrasonore est configuré pour lancer une mesure ultrasonore en direction du membre.

Dans un mode de réalisation, le transducteur ultrasonore émet un signal ultrasonore.

Dans un mode de réalisation, le transducteur ultrasonore comprend au moins un capteur ultrasonore, chaque capteur ultrasonore étant configuré pour émettre un signal ultrasonore et recevoir un signal réfléchi par le membre.

Dans un mode de réalisation, le transducteur ultrasonore comprend plusieurs capteurs ultrasonores, notamment pour couvrir une plus grande zone et offrir une résolution plus fine grâce à l'analyse combinée des signaux provenant de plusieurs capteurs.

Dans un mode de réalisation, chaque capteur ultrasonore comprend un dispositif piézoélectrique, notamment en céramique.

Dans un mode de réalisation, le signal émis est un signal pulsé à fréquence constante. Le capteur alterne entre émission et réception pour analyser des zones spécifiques à une certaine profondeur.

Dans un mode de réalisation, la fréquence est comprise entre 1 et 50 MHz, notamment 8 et 15 MHz.

Dans un mode de réalisation, le transducteur ultrasonore est configuré pour balayer le membre de l'utilisateur pour localiser une artère de l'utilisateur, notamment en analysant la vitesse du sang, les artères ayant généralement un flux pulsatile plus rapide que les veines.

Dans un mode de réalisation, lorsque le dispositif est une montre, le transducteur ultrasonore est configuré pour détecter l'artère radiale.

Dans un mode de réalisation, le transducteur ultrasonore est configuré pour collecter les signaux Doppler des flux sanguins des artères et des micro-emboles circulant dans les artères.

Dans un mode de réalisation, l'unité de contrôle est configurée pour recevoir le signal Doppler et pour analyser le signal Doppler basé sur la différence de fréquence entre le signal émis et le signal réfléchi, en général entre 20 Hz à 50 kHz.

Dans un mode de réalisation, l'unité de contrôle est en outre configurée pour déduire du signal Doppler la vitesse des globules rouges et des micro-emboles dans le sang circulant dans les artères.

Dans un mode de réalisation, le transducteur ultrasonore est disposé dans un bracelet du dispositif.

Dans un mode de réalisation, l'unité de contrôle est configurée pour recevoir le signal Doppler et configurée pour détecter au moins une micro-embole dans l'artère par analyse du signal Doppler.

Dans un mode de réalisation, le signal Doppler est envoyé par le dispositif vers le terminal externe ou le serveur distant pour analyse.

Dans un mode de réalisation, l'unité de contrôle est configurée pour détecter le passage d'une micro-embole par la détection d'une variation de l'amplitude dans le signal Doppler.

Dans un mode de réalisation, l'unité de contrôle est configurée pour détecter un pic d'intensité (appelée, HITS, pour « *high intensity transient signal » en anglais*) dans le signal Doppler, notamment par rapport à l'amplitude de référence du flux sanguin environnant, caractéristique d'un passage d'une micro-embole.

Dans un mode de réalisation, l'unité de contrôle est configurée pour émettre un signal d'alerte en cas de détection d'un débit de micro-emboles supérieur à un seuil d'alerte prédéterminé, par exemple un message d'alerte à l'utilisateur et/ou à une personne tierce.

Dans un mode de réalisation, le seuil d'alerte est compris entre 10 et 100 micro-emboles par minute détectées.

Dans un mode de réalisation, le membre est un poignet ou un bras de l'utilisateur.

Dans un mode de réalisation, le dispositif est une montre.

Dans un mode de réalisation, le dispositif est un tensiomètre.

Dans un mode de réalisation, le dispositif est une balance.

Dans un mode de réalisation, le dispositif comprend une batterie rechargeable configurée pour fournir de l'énergie au dispositif.

L'invention concerne également une méthode d'activation mise en oeuvre par une unité de contrôle d'un dispositif tel que décrit ci-dessus, comprenant les étapes de :
- activation du capteur physiologique et commande d'une génération d'un signal cardiaque ;
- réception et analyse du signal cardiaque ;
- en réponse à une détection d'une arythmie cardiaque dans l'analyse du signal cardiaque, activation du transducteur ultrasonore.

Dans un mode de réalisation, la méthode comprend en outre la commande d'une génération d'un signal Doppler par le transducteur ultrasonore.

Dans un mode de réalisation, la méthode comprend en outre la réception et l'analyse du signal Doppler par l'unité de contrôle.

Dans un mode de réalisation, la méthode comprend l'émission du signal Doppler vers un terminal externe ou un serveur distant pour analyse.

Dans un mode de réalisation, la méthode comprend en outre, en réponse à une détection d'une micro-embole, l'émission d'un signal d'alerte.

### Brève description des dessins

D'autres caractéristiques, détails et avantages apparaîtront à la lecture de la description détaillée ci-après, et à l'analyse des dessins annexés, sur lesquels :
- [Fig. 1] La figure 1 présente une représentation schématique d'un dispositif selon la présente description au contact d'un membre d'un utilisateur.
- [Fig. 2] La figure 2 présente deux signaux ECG (tension en ordonnée et temps en abscisse), présentant un rythme sinusal (a) et présentant une arythmie (b).
- [Fig. 3] La figure 3 présente une échographie Doppler comprenant un pic d'intensité caractéristique du passage d'une micro-embole
- [Fig. 4] La figure 4 présente une représentation schématique du dispositif et de son environnement.
- [Fig. 5] La figure 5 présente une vue de dessus du dispositif sous la forme d'une montre.
- [Fig. 6] La figure 6 présente une vue de dessous de la montre de la figure 5.
- [Fig. 7] La figure 7 présente une vue de dessous de la montre de la figure 5 selon un autre mode de réalisation.
- [Fig. 8] La figure 8 présente une vue en coupe transversale de la montre de la figure 5.
- [Fig. 9] La figure 9 présente une vue en perspective du dispositif sous la forme d'un tensiomètre.
- [Fig. 10] La figure 10 présente une vue en perspective du dispositif sous la forme d'une balance.
- [Fig. 11] La figure 11 présente un diagramme représentant une méthode d'activation d'un transducteur ultrasonore selon la description.

### Description détaillée des modes de réalisation

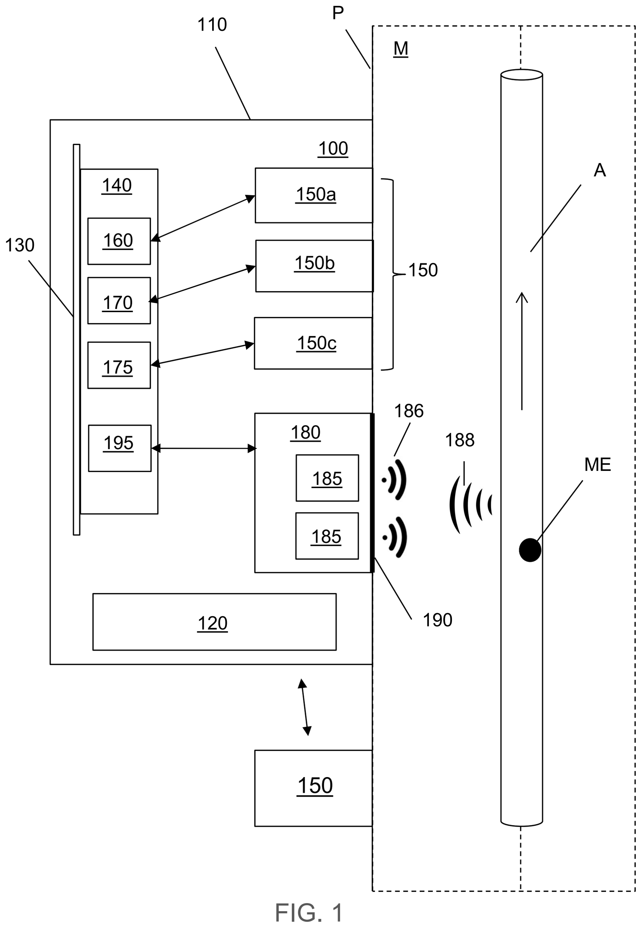

Un dispositif 100 est représenté sur la figure 1.

Le dispositif est notamment un dispositif portable. Par « portable » (*« wearable »* en anglais), on entend un dispositif qui est positionnable au contact d'un utilisateur et transportable aisément par cet utilisateur. En particulier, le dispositif est configuré pour être positionné sur la peau P d'un membre M de l'utilisateur, et notamment pour y être positionné en continu. Dans la suite de la description, par « en continu » il est entendu qui se déroule sans interruption sur une durée longue, par exemple au moins une heure, notamment au moins une journée complète. Le dispositif 100 peut toutefois être retiré de manière occasionnelle, par exemple pour la recharge. Comme cela sera décrit plus en détail par la suite, le membre M est par exemple un poignet ou un bras. Toutefois, la description n'est pas limitée à ces membres et s'applique à tout membre de l'utilisateur.

Le dispositif 100 comprend une enveloppe 110. L'enveloppe 110 forme une enceinte qui définit un volume interne apte à recevoir divers composants, tels que des composants électroniques, comme cela sera expliqué plus en détail ci-dessous. L'enveloppe 110 protège le volume interne notamment de la poussière, de l'eau, de l'humidité et des chocs. L'enveloppe 110 peut présenter une dimension transversale maximale inférieure à 30 cm, notamment inférieure à 10 cm.

Le dispositif 100 peut présenter une masse inférieure à 1 kg, notamment inférieure à 500 g, notamment inférieure à 250 g et notamment inférieure à 100g.

Le dispositif 100 peut comprendre une batterie 120, disposée dans le volume interne défini par l'enveloppe 110 ou dans le bracelet. La batterie 120 est configurée pour fournir de l'énergie au dispositif 100, et en particulier aux composants électroniques du dispositif 100. La batterie 120 est par exemple une pile ou une batterie rechargeable.

### UNITÉ DE CONTRÔLE

Le dispositif 100 peut comprend au moins une carte de circuit imprimé 130 (également appelée PCB pour « *printed circuit board »* en anglais). Chaque carte de circuit imprimé est une carte mince et rigide contenant un circuit imprimé. La ou les cartes de circuit imprimée 130 comprennent une unité de contrôle 140, illustrée schématiquement sur la figure 1. D'autres composants peuvent également être montés sur la ou les cartes de circuit imprimé 130.

L'unité de contrôle 140 est utilisée pour contrôler l'électronique embarquée du dispositif 100. L'unité de contrôle 140 peut, par exemple, inclure ou partiellement inclure un module PPG, un module ECG, un module oscillométrique et un module Doppler comme cela sera expliqué ci-dessous.

### CAPTEUR PHYSIOLOGIQUE

Le dispositif 100 comprend un capteur physiologique 150 configuré pour générer un signal cardiaque caractéristique d'une activité cardiaque de l'utilisateur. Un signal caractéristique de l'activité cardiaque de l'utilisateur est une mesure physiologique qui reflète l'activité électrique et/ou mécanique du cœur, notamment pour surveiller ou analyser le fonctionnement du système cardiovasculaire. Un signal caractéristique de l'activité cardiaque de l'utilisateur est par exemple un électrocardiogramme (ECG), un Photopléthysmogramme (PPG), un Phonocardiogramme (PCG), une mesure d'impédance cardiographique (ICG), un signal oscillométrique, un signal d'impédancemétrie (IPG), un signal de ballistocardiogramme (BCG).

Le capteur physiologique 150 génère typiquement un signal cardiaque en continu. Cela permet une surveillance sans interruption, y compris la nuit lorsque le dispositif 100 est porté la nuit.

Dans un mode de réalisation, le capteur physiologique 150 est disposé dans le volume interne défini par l'enveloppe 110. Alternativement, le capteur physiologique 150 peut être positionné dans un bracelet permettant d'attacher l'enveloppe 110 au membre M. En variante encore, le capteur physiologique 150 peut être disposé à l'extérieur du volume interne défini par l'enveloppe 110 et connecté à l'unité de contrôle 140 par un fil électrique ou de manière sans-fil, par exemple par Bluetooth.

### Capteur optique

Dans un mode de réalisation, le capteur physiologique 150 est un capteur optique 150a. Le capteur optique est généralement un capteur PPG (photopléthysmographie), comprenant au moins une source de lumière, par exemple une LED (diode électroluminescente), pour émettre un signal lumineux et au moins récepteur de lumière, par exemple une photodiode, pour recevoir le signal lumineux.

Le capteur optique 150a est connecté à un module PPG 160, qui peut être monté sur la carte de circuit imprimé 130. Le module PPG 160 est configuré pour générer les instructions d'émission pour les LED et pour récupérer les signaux électriques des photodiodes.

Dans un mode de réalisation, le capteur optique 150a, via le module PPG 160, est configuré pour effectuer une mesure du signal cardiaque en continu. A cet effet, la source de lumière émet un signal lumineux en continu. Le capteur optique est alors configuré pour surveiller en temps réel l'activité cardiaque de l'utilisateur.

En variante, le capteur optique 150a est configuré pour effectuer une mesure du signal cardiaque de manière périodique, notamment à une fréquence comprise entre une mesure toutes les minutes et une mesure toutes les 2 minutes, voire toutes les 5 minutes, voire toutes les 10 minutes.

### Capteur ECG

En variante ou en complément, le capteur physiologique 150 est un capteur ECG 150b, configuré pour mesurer un électrocardiogramme (« ECG ») de l'utilisateur.

Le capteur ECG 150b est configuré pour récupérer les signaux électriques générés par le corps humain. En particulier, le capteur ECG 150b comprend un ensemble d'électrodes (appelées électrodes ECG par la suite) et est connecté à un module ECG 170, qui peut être monté sur la carte de circuit imprimé 130 et auquel les électrodes ECG sont électriquement connectées. Par électrode, on entend une partie conductrice capable de recevoir un courant ou une tension électrique. La pièce peut être constituée d'un matériau conducteur ou comporter un revêtement conducteur. Par « conducteur », on entend « conducteur d'électricité ».

Dans un mode de réalisation, le dispositif 100 peut comprendre uniquement deux électrodes ECG. En variante, le dispositif 100 peut comprendre une troisième électrode ECG.

Le module ECG 170 est configuré pour récupérer des signaux électriques du corps humain et pour générer, après traitement, un signal d'électrocardiogramme ou une donnée représentative d'une information sur l'électrocardiogramme. A cet effet, le module ECG 170 peut mettre en œuvre un algorithme d'apprentissage profond pour le traitement et l'analyse du signal ECG.

Le capteur ECG 150b présente d'avantage de contrainte en termes de positionnement sur le corps, puisqu'il est nécessaire d'effectuer une boucle pour générer un signal ECG.

Dans un mode de réalisation, les électrodes ECG sont arrangées sur l'enveloppe 110.

En variante, au moins une électrode ECG est à l'écart de l'enveloppe 110 et reliée de manière filaire ou sans-fil à l'unité de contrôle 140.

Pour mesurer un ECG en continu, le capteur ECG 150b peut être un capteur attaché au torse.

### Capteur oscillométrique

En variante ou en complément, le capteur physiologique 150 est un capteur oscillométrique 150c, configuré pour réaliser une mesure oscillométrique de l'utilisateur.

Un capteur oscillométrique est notamment utilisé pour mesurer la pression artérielle d'un utilisateur de manière non invasive, par détection de variations de pression dans un brassard gonflable placée autour du bras ou du poignet.

Le capteur optique 150c est connecté à un module oscillométrique 175, qui peut être monté sur la carte de circuit imprimé 130. Le module oscillométrique 175 est configuré pour générer les instructions de mesure oscillométrique.

Le module oscillométrique 175 est notamment configuré pour détecter les oscillations de pression causées par les battements du cœur dans l'artère.

### TRANSDUCTEUR ULTRASONORE

Le dispositif 100 comprend un transducteur ultrasonore 180 configuré pour générer un signal Doppler caractéristique d'une circulation sanguine dans le membre M. En particulier, le transducteur ultrasonore 180 est configuré pour lancer une mesure ultrasonore en direction du membre M. Le transducteur ultrasonore peut être également appelé sonde Doppler.

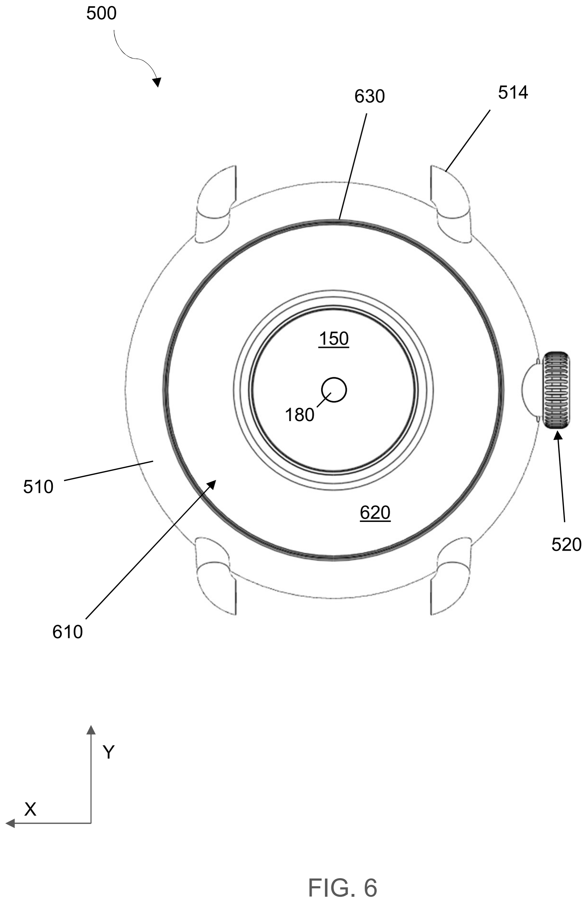

Sur la figure 1, le transducteur ultrasonore 180 est disposé dans le volume interne défini par l'enveloppe 110. Toutefois, alternativement le transducteur ultrasonore 180 peut être positionné dans un bracelet permettant d'attacher l'enveloppe 110 au membre M (comme présenté par exemple dans le document US20240130617A1, Figure 6 et paragraphe 183).

A cet effet, le transducteur ultrasonore 180 comprend au moins un capteur ultrasonore 185. Chaque capteur ultrasonore 185 est configuré pour émettre un signal ultrasonore 186 et recevoir un signal réfléchi 188 par le membre M et notamment par le système cardiovasculaire du membre, c'est-à-dire essentiellement par une ou plusieurs artères présentes localement dans le membre M. Dans un mode de réalisation, le transducteur ultrasonore 180 comprend plusieurs capteurs ultrasonores 185, notamment pour couvrir une plus grande zone dans le membre M et offrir une résolution plus fine grâce à l'analyse combinée des signaux provenant de plusieurs capteurs ultrasonores.

Le ou les capteurs ultrasonores 185 sont choisis parmi les capteurs ultrasonores capacitifs micro-usinés (CMUT), les capteurs ultrasonores piézoélectriques micro-usinés (PMUT) ou les capteurs piézoélectriques. Dans un mode de réalisation, les capteurs ultrasonores sont de type capacitifs micro-usinés (CMUT) ou ultrasonores piézoélectriques micro-usinés (PMUT). Dans un mode de réalisation, les capteurs ultrasonores sont de type capacitif micro-usiné (CMUT).

Selon un mode de réalisation, le ou les capteurs ultrasonores sont constitués d'au moins une barrette comprenant au moins deux éléments. Dans un mode de réalisation, les capteurs ultrasonores sont des éléments isolés.

Le signal émis par chaque capteur ultrasonore 185 peut être un signal pulsé à fréquence constante. Chaque capteur ultrasonore 185 est configuré pour alterner entre émission d'un signal et réception d'un signal, notamment pour analyser des zones spécifiques à une certaine profondeur dans le membre M. Chaque capteur ultrasonore 185 est configuré pour émettre un signal sonore à une fréquence comprise entre 1 et 50 MHz, notamment entre 8 et 15 MHz.

En variante, le transducteur ultrasonore 180 est configuré pour réaliser l'émission et l'acquisition des signaux Doppler en continu.

Le transducteur ultrasonore 180 est configuré pour générer le signal Doppler basé sur la différence de fréquence entre le signal émis et le signal réfléchi, dû à l'effet Doppler. La différence de fréquence est notamment comprise entre 20 Hz à 20 kHz.

Le transducteur ultrasonore 180 est configuré pour émettre un signal Doppler avec des faisceaux focalisés (ou « beamformés ») ou des faisceaux non focalisés, notamment en onde plane ou en onde sphérique.

Dans un mode de réalisation, le transducteur ultrasonore 180 est configuré pour balayer le membre M de l'utilisateur pour localiser une artère A de l'utilisateur. En particulier, le transducteur ultrasonore 180 est configuré pour modifier la direction d'émission des signaux ultrasonores, par exemple par déplacement d'un ou plusieurs éléments piézo-électriques. La localisation de l'artère A s'effectue notamment en analysant la vitesse du sang dans les différents vaisseaux détectés, les artères ayant généralement un flux pulsatile plus rapide que les veines.

Pour entrer en contact la peau du membre, le transducteur ultrasonore 180 peut comprend une interface 190. Dans un mode de réalisation, l'interface 190 comprend un matériau souple, par exemple du silicone, arrangé de sorte à être au contact de la peau, pour améliorer le couplage acoustique avec la peau.

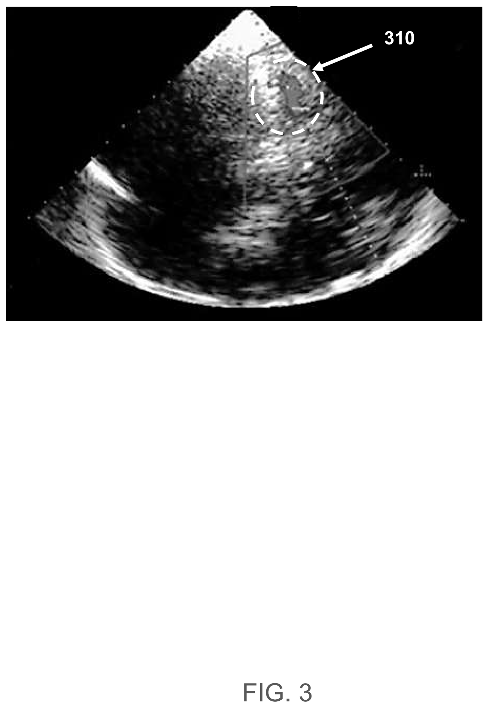

Le transducteur ultrasonore 180 permet notamment de générer des images Doppler telle que représentée en Figure 3 et qui sera détaillée par la suite.

### ARCHITECTURE

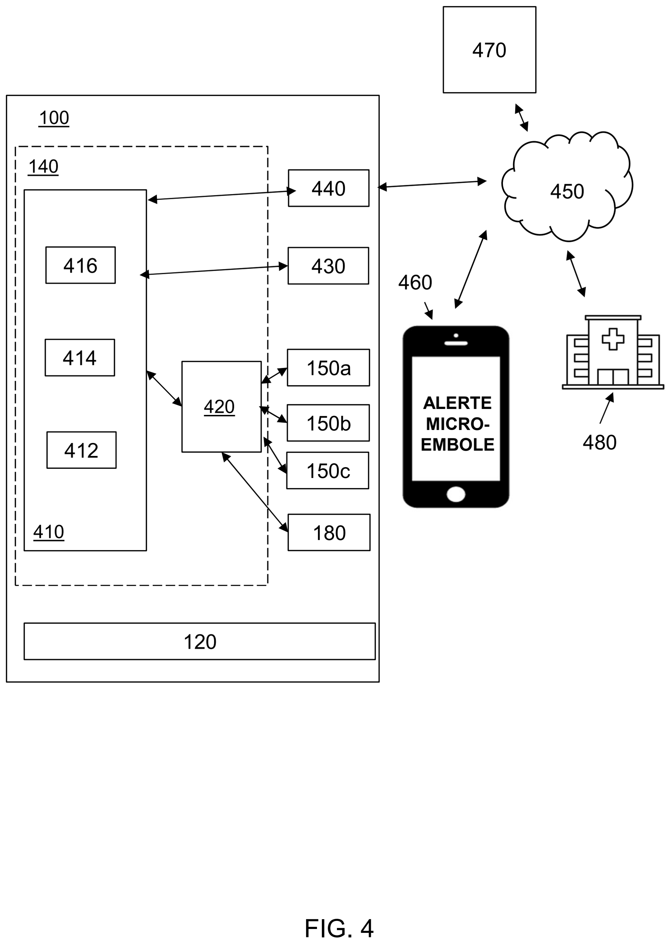

La figure 4 illustre un schéma de l'architecture d'un dispositif 100 tel que décrit et de son environnement.

L'unité de contrôle 140 comprend une circuiterie de contrôle 410 comprenant un processeur 412, une mémoire 414 et une interface I/O 416 (« In/Out » en anglais ou « Entrée/Sortie » en français) pour communiquer avec les autres composants.

La mémoire 414 stocke des programmes, instructions ou autres permettant à la fois la navigation sur le dispositif 100 ainsi que la prise des mesures (algorithmes notamment). La mémoire 414 en particulier se décompose en une mémoire volatile, de type RAM, et une mémoire non-volatile, de type flash (ou ROM ou SSD).

L'unité de contrôle 140 comprend le module optique 160, le module ECG 170, le module oscillométrique 175 et/ou le module Doppler 195. L'unité de contrôle 140 est notamment configurée pour commander l'activation et la mesure par le ou les capteurs physiologiques 150 et/ou le transducteur ultrasonore 180.

L'unité de contrôle 140 est notamment arrangée sur une carte de circuit imprimé, dite carte de circuit imprimée principale 130. L'unité de contrôle 140 peut être constituée de plusieurs sous unités, arrangées sur le circuit imprimé principal 130 et sur d'autres cartes de circuit imprimé.

L'unité de contrôle 140 comprend typiquement un module d'interface 420 faisant interface entre le ou les capteurs physiologiques 150, le transducteur ultrasonore 180 et l'interface I/O 416 de la circuiterie de contrôle 410. Le module d'interface 420 comprend notamment des ADC, des filtres, des amplificateurs, etc.

Le dispositif 100 comprend en outre l'affichage 430, qui communique avec l'interface I/O 416.

La batterie 120 est configurée pour alimenter les différents composants en énergie électrique du dispositif 100, par exemple une pile ou une batterie rechargeable. La batterie 120 est configurée pour alimenter notamment l'unité de contrôle 140, l'affichage 430, le ou les capteurs physiologiques 150 et le transducteur ultrasonore 180.

### CONNECTIVITÉ

Toujours en référence à la figure 4, le dispositif 100 comprend un module de communication sans fil 440, tel qu'un module Bluetooth ou Bluetooth Low Energy ou un module Wi-Fi ou un module cellulaire (GSM, 2G, 3G,4G, 5G, Sigfox, etc.), qui lui permet de communiquer, via un réseau de communication 450, de manière bidirectionnelle avec au moins un terminal externe 460, tel qu'un smartphone. Le terminal externe 460 peut lui aussi communiquer (de manière bidirectionnelle) avec le serveur distant 470 pour le stockage et le traitement des données. En alternative ou en complément, le module de communication sans fil 440 peut communiquer directement avec le serveur distant 470, par exemple via le réseau cellulaire ou via un réseau Wi-Fi.

Via le réseau de communication 450, le terminal externe 460 ou le serveur distant 470 peuvent communiquer avec un service d'assistance 480.

### UNITÉ DE CONTRÔLE - COMPLÉMENTS

### Analyse du signal cardiaque

L'unité de contrôle 140 est configurée pour recevoir un signal cardiaque du (ou des) capteur physiologique 150. Le signal cardiaque est notamment un signal optique et/ou un signal ECG.

L'unité de contrôle 140 est configurée pour analyser le signal cardiaque reçu. En particulier, l'unité de contrôle 140 est configurée pour caractériser le signal cardiaque reçu, notamment pour détecter la suspicion de la présence d'une arythmie cardiaque. La suspicion de présence est une probabilité de présence d'une arythmie dans le signal cardiaque supérieure à un seuil prédéterminé. Le seuil prédéterminé est par exemple égal à une probabilité de 50%. L'arythmie détectée est en particulier une fibrillation atriale. L'analyse du signal cardiaque par l'unité de contrôle 140 du dispositif 100 lui-même permet d'économiser de la batterie et d'avoir une analyse quelle que soit l'état de connectivité du dispositif 100 avec le terminal externe 460 ou le serveur distant 470 et sans délai lié à l'envoi du signal cardiaque vers le terminal externe 460 ou le serveur distant 470 (pour que le signal y soit analysé) et à la réception de l'analyse.

En particulier, l'unité de contrôle 140 peut être configurée pour mettre en œuvre un pré-traitement du signal cardiaque reçu. Le pré-traitement comprend par exemple un rééchantillonnage du signal pour le mettre à une fréquence qui peut être traitée par la suite et/ou un filtrage pour éliminer les bruits (mouvements, artefacts, etc.).

L'unité de contrôle 140 peut être configurée pour mettre en œuvre un traitement du signal reçu. Le traitement comprend par exemple la détection des pics de pulsation et l'analyse de l'intervalle entre ces pics (dit intervalle RR, du nom du pic R dans le complexe QRS de l'ECG).

L'identification d'arythmie à l'aide d'un signal cardiaque, notamment un signal ECG , un signal PPG, un signal oscillométrique, un signal BCG ou un signal IPG est connue et ne sera pas décrite en détail. Par exemple, le document WO2024194199 décrit un algorithme embarquable sur une montre pour détecter des arythmies. Par exemple, le document Pereira (Pereira, T., Tran, N., Gadhoumi, K. et al. Photoplethysmography based atrial fibrillation detection: a review. npj Digit. Med. 3, 3 (2020). https://doi.org/10.1038/s41746-019-0207-9) présente une revue de la détection de fibrillation atriale à l'aide de signaux PPG.

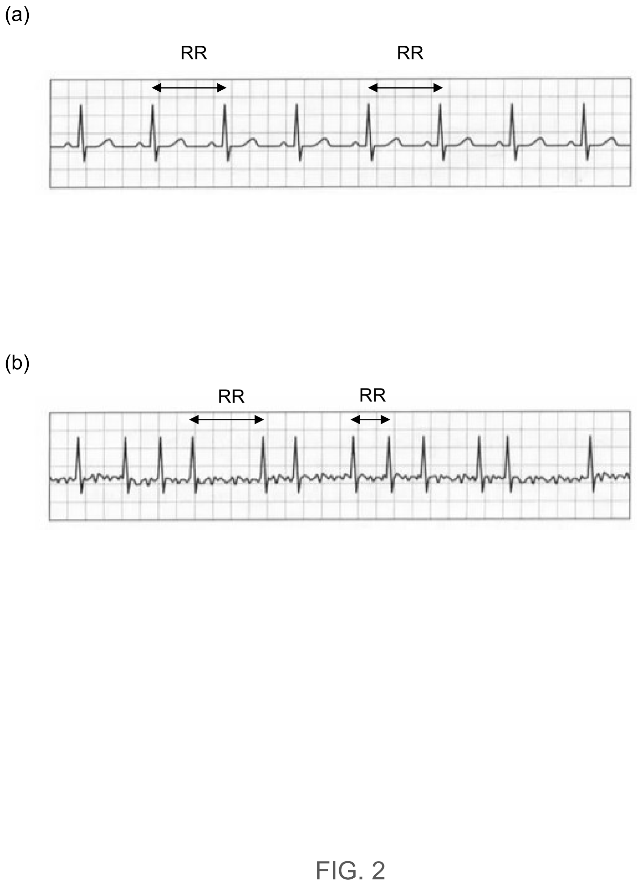

La figure 2 illustre un signal ECG dans un cas (a) où les intervalles RR sont réguliers et le signal cardiaque est dit « normal » ou présentant un « rythme sinusale ». Dans le cas (b), les intervalles RR sont irréguliers, caractéristique d'une activité cardiaque irrégulière. Le signal cardiaque présente alors une arythmie, qui peut notamment est une fibrillation atriale

L'unité de contrôle 140 peut être configurée pour mettre en œuvre une classification du signal cardiaque, notamment sur la base du signal prétraité et/ou traité. La caractérisation du signal cardiaque peut être mise en œuvre à l'aide d'un algorithme d'apprentissage profond (« *deep learning* »), par exemple de type classifieur. Les résultats de la classification peuvent être par exemples les classes suivantes : « mauvaise qualité de signal », « fibrillation atriale », « autre arythmie », « rythme sinusale », « autre ».

Dans un mode de réalisation, l'unité de contrôle 140 peut être configurée pour mettre en œuvre une régression. L'unité de contrôle 140 peut être configurée pour attribuer une valeur au signal analysé, notamment une valeur qui représente la suspicion de présence d'une arythmie, autrement dit la probabilité de présence d'une arythmie.

L'unité de contrôle 140 est configurée pour analyse en continu le signal cardiaque reçu. En particulier, l'analyse peut se faire au moyen d'une fenêtre temporelle glissante sur le signal cardiaque reçu, par exemple une fenêtre glissante de 30s. Cette méthode permet d'obtenir une analyse en temps réel : l'arythmie peut ainsi être détectée en temps réel, c'est-à-dire instantanément par rapport à son occurrence. Alternativement, l'analyse peut se faire au moyen de portions successives de signaux, par exemple des portions successives de 30s. Cette méthode est moins coûteuse en énergie mais crée un retard compris entre 0 et 30s pour l'analyse. L'analyse peut également se faire sur un intervalle de temps plus important, par exemple au moins une minute, pour détecter plusieurs fois une arythmie dans le signal cardiaque durant cet intervalle de temps pour confirmer la présence d'une arythmie.

### Activation du transducteur ultrasonore

L'unité de contrôle 140 est configurée pour activer le transducteur ultrasonore 180 en réponse à une détection d'une arythmie cardiaque dans l'analyse du signal cardiaque. Autrement dit, sauf activation expresse liée à d'autres raisons (par exemple une mesure volontaire de l'utilisateur), dans le cadre de la présente description, le transducteur ultrasonore 180 est activé et effectue des mesures uniquement lorsqu'une arythmie est détectée dans le signal cardiaque reçu et analysé par l'unité de contrôle 140.

L'activation se fait sans délai après détection d'une arythmie afin d'optimiser les chances d'obtenir un signal Doppler caractéristique de la circulation sanguine dans le membre concomitamment ou très peu de temps (le moins possible) après la survenance de l'arythmie.

Lorsque le transducteur ultrasonore 180 est activé, le transducteur ultrasonore 180 est configuré pour lancer une mesure de manière instantanée ou quelques secondes après l'activation, de sorte que le signal Doppler généré par le transducteur ultrasonore est concomitant au signal cardiaque comprenant une arythmie ou généré quelques secondes après celui-ci. Par quelques secondes, on entend inférieur à 10 secondes.

L'unité de contrôle 140 est configurée pour recevoir le signal Doppler généré par le transducteur ultrasonore 180. L'unité de contrôle 140 est en outre configurée pour déduire du signal Doppler la vitesse des globules rouges dans le sang circulant dans l'artère A.

Dans un mode de réalisation, l'unité de contrôle 140 est en outre configurée pour analyser le signal Doppler afin de détecter au moins une micro-embole ME dans l'artère A. En particulier, l'unité de contrôle 140 est configurée pour mesurer une quantité de micro-embole ME par unité de temps, par exemple par minute. Cette analyse embarquée permet au dispositif 100 d'être autonome dans la détection d'embolie ou du micro-embole, sans qu'il soit nécessaire que le dispositif 100 communique avec le terminal externe 460 ou le serveur distant 470.

Alternativement, le signal Doppler est envoyé par le dispositif 100 vers le terminal externe 460 ou le serveur distant 470 pour y être analysé afin de profiter de capacité de calcul accrue comparativement à l'unité de contrôle 140 embarquée.

En particulier, l'analyse pour détecter le passage des micro-emboles ME peut comprendre la détection d'une variation de l'amplitude dans le signal Doppler. L'analyse comprend alors la détection d'un pic d'intensité (appelée, HITS, pour « *high intensity transient signal »* en anglais ou *« signal transitoire de haute intensité »* en français) dans le signal Doppler, par rapport à l'amplitude de référence du flux sanguin environnant, caractéristique d'un passage d'au moins une micro-embole ME. Un tel pic 310 est illustré sur la figure 3 par exemple qui représente une échographie Doppler obtenue par une transducteur ultrasonore 180. L'amplitude des micro-emboles est généralement supérieure de 10 à 20 dB à celle des signaux du flux sanguin.

L'analyse peut être constituée, sans exhaustivité, au moyen de techniques avancées telles que la transformée de Fourier rapide (FFT), les transformées en ondelettes et le filtrage par seuil améliorant la détection des signaux emboliques en isolant les pics transitoires de forte amplitude dans le spectre Doppler et/ou la classification des signatures des signaux emboliques, de combinaison d'analyse spectrale et de techniques de regroupement de signaux permettant d'identifier les signatures emboliques, de réseaux neuronaux convolutionnels (CNN) développés sur mesure pour l'analyse de spectrogrammes basée sur l'image, de modèles en apprentissage automatique (ou « machine learning en anglais) entraînés pour la classification des signaux Doppler des micro-emboles, ou de méthodes combinant deux ou plusieurs des méthodes précédentes.

L'analyse du signal Doppler peut être effectuée avec un délai ou un retard, du moment que le signal Doppler ait été acquis le plus rapidement possible après la détection d'arythmie.

Dans un mode de réalisation, l'unité de contrôle 140 est configurée pour laisser le transducteur ultrasonore 180 actif pendant un intervalle de temps prédéterminé, par exemple compris entre 10 secondes et 5 minutes, ou compris entre 10 secondes et 1 minute. L'unité de contrôle 140 est configurée pour désactiver le transducteur ultrasonore 180 à la fin de cet intervalle de temps, pour économiser la batterie 120. Ainsi, dans un mode de réalisation, le signal Doppler, à l'inverse du signal cardiaque, n'est pas reçu en continu par l'unité de contrôle 140 mais uniquement pendant des intervalles de temps limité, juste après détection d'une arythmie dans le signal cardiaque.

La fibrillation auriculaire (ou « Afib ») est un facteur de risque majeur d'accident vasculaire cérébral (« AVC ») en raison de la formation de caillots sanguins dans le cœur, qui peuvent migrer vers le cerveau. Lors de l'Afib, les oreillettes ne se contractent pas efficacement. Cela provoque une stagnation du sang qui favorise la formation de caillots sanguins. Ces caillots sanguins peuvent être éjectés du cœur lorsque le cœur pompe. Ce caillot éjecté devient alors une micro-embole. Si une micro-embole atteint les artères cérébrales, il peut obstruer le flux sanguin et causer un AVC ischémique. L'unité de contrôle 140 permet donc de détecter une telle micro-embole à la suite d'une détection d'un évènement d'arythmie cardiaque, notamment de fibrillation auriculaire. La détection en continu, voire en temps réel de l'arythmie, permet une surveillance à tout moment.

### Emission d'une alerte

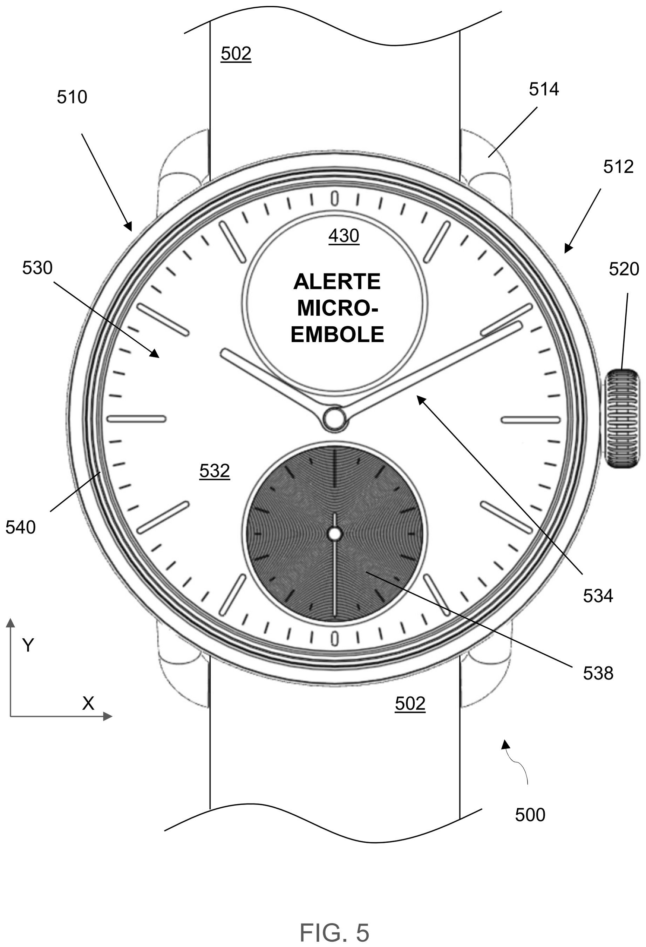

L'unité de contrôle 140 est configurée pour émettre un signal d'alerte en cas de détection d'un débit de micro-emboles ME supérieur à un seuil d'alerte combinée à la détection d'une arythmie. Le seuil d'alerte est par exemple compris entre 10 et 100 micro-emboles par minute détectées. Le signal d'alerte peut être un message d'alerte affiché sur un affichage 430 du dispositif 100, comme illustré plus en détail sur le dispositif 100 sous forme de montre 500 de la Figure 5. En variante ou en complément, le signal d'alerte est un message d'alerte envoyé à l'utilisateur, par exemple sur un terminal externe 460 de l'utilisateur, tel qu'un smartphone, comme illustré en Figure 4. En variante ou en complément, le signal d'alerte peut être un message d'alerte envoyé à une personne tierce, par exemple un proche ou un personnel médical d'un service d'assistance 480, qui suit l'utilisateur à distance.

L'émission d'une alerte permet une réaction de l'utilisateur rapide face au risque d'AVC et permet notamment une intervention médicale rapide, ce qui est peut être crucial pour prévenir les dommages causés par un AVC.

Dans un mode de réalisation, l'unité de contrôle 140 est configurée pour émettre également un signal d'alerte à la détection d'une arythmie, sans détection d'une micro-embole ME, notamment pour permettre un accompagnement médical de l'utilisateur.

### MONTRE

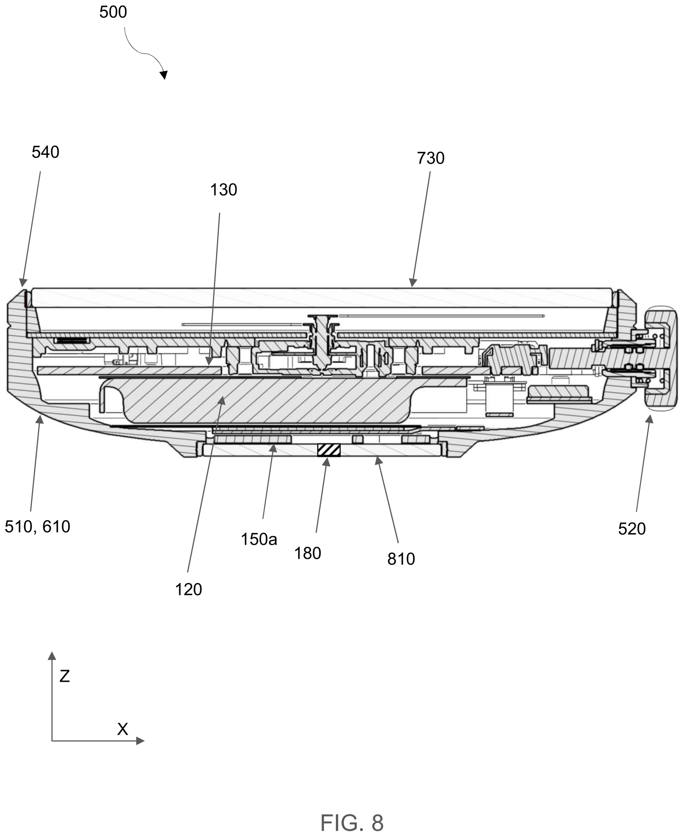

Dans un mode de réalisation, le dispositif portable 100 est configuré pour être placé sur le poignet d'un utilisateur. Dans ce mode de réalisation, le dispositif 100 est par exemple une montre 500, comme illustrée sur les figures 5 à 8. La montre 500 peut comprendre un bracelet 502. Le dispositif peut également être un tracker d'activité.

En particulier, les figures 5 à 8 illustrent une montre 500 électronique, de type hybride, avec un cadran, des aiguilles mécaniques, et éventuellement un affichage intégré au cadran. Un repère standard (XYZ) est représenté sur ces figures.

Dans une variante non représentée, la montre peut être une montre non hybride sans aiguilles mécaniques mais avec un écran d'affichage.

L'enveloppe 110 du dispositif 100 peut comprendre un boîtier 510 et un fond de boîtier 610 illustrés à la figure 6.

Lorsque le dispositif 100 est une montre, le boîtier 510 peut également être appelé carrure (« case » en anglais). Le boîtier 510 peut comprendre une paroi latérale 512 qui est généralement visible lorsque la montre 500 est portée au poignet. Le boîtier 510 peut comprendre des pattes 514 pour la fixation d'un bracelet (non représenté sur les figures). En particulier, le boîtier 510 peut comprendre deux paires de pattes 514, de part et d'autre du boîtier 510. Le boîtier 510 peut comprendre une pluralité de pièces.

La montre 500 comprend en outre une couronne 520 (*« crown »* en anglais) faisant saillie à travers le boîtier 510. Comme on peut le voir sur les figures 5 à 7, la couronne 520 fait saillie orthogonalement à l'axe Z, le long d'un axe X.

Dans les modes de réalisation illustrés, la montre 500 comprend une couronne 520 unique. En variante non représentée, la montre 500 peut comprendre une pluralité de couronnes 520. La couronne 520 présente notamment un rôle d'interface utilisateur entre la montre 500 et l'utilisateur. En particulier, la couronne 520 peut être utilisée par l'utilisateur pour régler l'heure ou la date, pour naviguer dans le menu, et/ou pour lancer l'enregistrement d'une activité, etc. La couronne 520 peut être un bouton poussoir et/ou une roue rotative.

Le fond de boîtier 610 est la face arrière de la montre 500. Le fond 610 est configuré pour être au moins partiellement en contact avec la peau du poignet de l'utilisateur. Dans un mode de réalisation, le fond 610 peut comprendre au moins en partie le ou les capteurs physiologiques 150, tel qu'un capteur optique 150a ou un capteur ECG 150b. Par exemple, le fond peut comprendre un élément annulaire 620. L'élément annulaire 620 peut entourer notamment le capteur optique 150a et le transducteur ultrasonore 180.

Dans un mode de réalisation illustré sur la figure 6, le boîtier 510 et le fond de boîtier 610 sont deux pièces distinctes. Dans ce mode de réalisation, le boîtier 510 et le fond 610 peuvent être séparés par un joint 630.

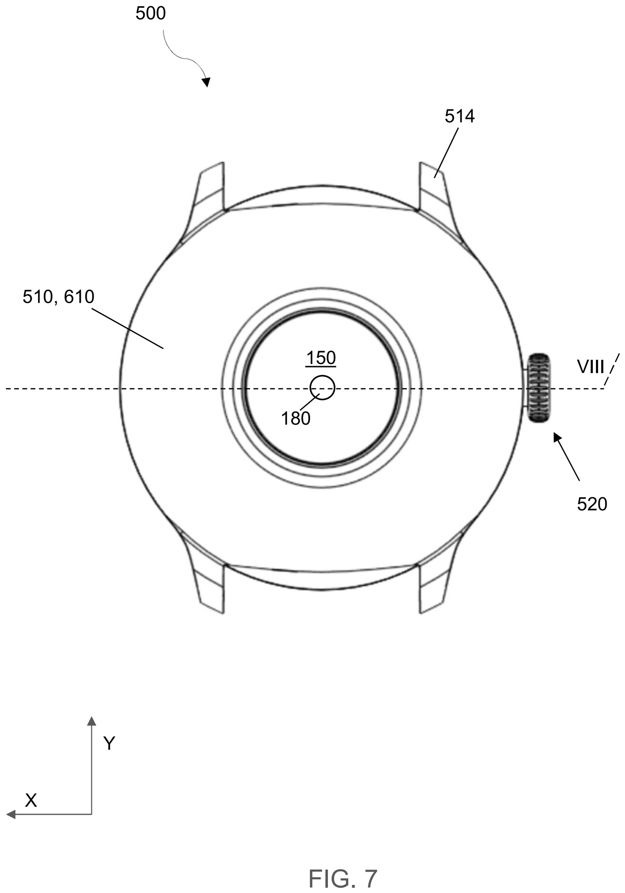

Dans une variante illustrée sur la figure 7, le boîtier 510 et le fond 610 sont solidaires l'un de l'autre. Dans ce mode de réalisation, le boîtier 510 et le fond 610 constituent une seule pièce mécanique.

La montre 500 peut également comprendre une glace 530 (« *glass* » ou *« crystal »* en anglais et usuellement appelé « verre » en horlogerie), typiquement montée sur le boîtier 510, de sorte que la glace 530 soit fixe. La glace 530 peut comprendre un verre protecteur typiquement transparent et peut être fabriqué en verre, en céramique, en plastique ou en tout autre matériau transparent. Le contour de la glace 530 est ici typiquement de forme circulaire.

Dans le cas d'une montre hybride, sous la glace 530, la montre 500 comprend en outre un cadran 532 avec des aiguilles physiques 534. Le cadran 532 peut également accueillir l'affichage 430, ici formé par un écran (par exemple, avec une ouverture dans le cadran qui permet à un écran positionné juste en dessous du cadran d'être visible), qui occupe, par exemple, un petit espace en dessous ou à l'intérieur du cadran 532. L'affichage 430 est notamment configuré pour afficher les messages d'alertes destiné à l'utilisateur.

La montre 500 peut également comprendre un cadran supplémentaire 538 pour afficher, par exemple, le nombre quotidien de pas effectués par l'utilisateur ou un autre indicateur de quantité d'activité physique. Le verre 530 protège ces parties et permet de les voir à travers.

Dans le cas d'une *« smartwatch* » de type Apple Watch^{™}, non représentée sur les figures, sous la glace 530, la montre 500 comprend un écran digital qui occupe une largeur proche de la largeur de la montre 500. Dans un mode de réalisation, l'écran peut afficher des aiguilles. La glace 530 est alors le verre protecteur de l'écran.

La montre 500 peut également comprendre une lunette 540 (*« bezel* » en anglais), montée sur le boîtier 510. La lunette 540 est positionnée autour du verre 530 (radialement externe au verre autour de la direction Z). Dans les modes de réalisation illustrés, la lunette 540 est montée de manière fixe par rapport au boîtier 510. Dans un mode de réalisation non représenté, la lunette 540 peut être montée de manière rotative par rapport au boîtier 510.

### Capteur optique

Le capteur physiologique 150 peut être un capteur optique 150a. Le capteur optique 150a est placé sur le fond de boîtier 610. Le capteur optique 150a est généralement un capteur PPG (photopléthysmographie), comprenant des LED pour émettre de la lumière et des photodiodes pour récupérer la lumière. Le capteur optique 150a peut être placé derrière une lentille 810, telle qu'une lentille en verre, qui fait interface avec la peau du poignet. Le document PCT/EP2021/058955, au nom de Withings™, et incorporé par référence, décrit en détail un mode de réalisation du capteur optique, que l'on retrouve notamment sur la Withings ScanWatch^{™} et la Withings ScanWatch Light^{™}. Le document EP24192447.1, au nom de Withings™, et incorporé par référence, décrit en détail un autre mode de réalisation du capteur optique, que l'on retrouve notamment sur la Withings ScanWatch 2^{™}.

### Capteur ECG

En variante ou en complément, le capteur physiologique 150 peut être un capteur ECG 150b. Le capteur ECG 150b comprend au moins deux électrodes ECG.

La première électrode ECG, est située sur le fond 610 de manière à être en contact avec la peau du poignet de l'utilisateur sur lequel il porte la montre 500. La première électrode ECG est connectée électriquement au module ECG 170. Dans un mode de réalisation, le fond de boîtier 610 est la première électrode ECG. Dans ce mode de réalisation, le fond de boîtier 610 est constitué d'un matériau conducteur d'électricité, tel que le métal.

Dans une variante, la première électrode ECG est disposée sur la lentille 810, par exemple avec un revêtement métallique.

La première électrode ECG peut entourer au moins partiellement le capteur optique 150b.

La deuxième électrode ECG, peut être disposée sur la lunette 540, de sorte que l'utilisateur peut toucher n'importe quelle partie de la lunette 540 pour effectuer un ECG. Par partie de la lunette 540, on entend toute partie de la surface de la lunette accessible à l'utilisateur par le toucher. Dans ce mode de réalisation, la couronne 520 n'est pas une électrode ECG.

Dans une variante, la deuxième électrode ECG est disposée sur la couronne 520.

Dans un mode de réalisation, le capteur ECG 150b peut comprendre une troisième électrode ECG, par exemple disposée sur le fond de boîtier 610.

### Capteur oscillométrique

En variante ou en complément, le capteur physiologique 150 peut être un capteur oscillométrique 150c. Le capteur oscillométrique 150c comprend par exemple sous la forme d'un bracelet avec un airbag gonflable. Un tel capteur est par exemple décrit dans les documents WO2024140132A1 et US20200345248A1.

### Transducteur ultrasonore

Le transducteur ultrasonore 180 peut comprendre un capteur ultrasonore miniaturisé. Par miniaturisé, on entend que la dimension transversale maximale du capteur ultrasonore est inférieure à 5 mm, notamment inférieur à 2 mm. Le capteur ultrasonore est notamment un émetteur-récepteur piézoélectrique.

Dans un mode de réalisation, le transducteur ultrasonore 180 est arrangé sur le fond de boîtier 610 pour être en contact avec la peau de l'utilisateur lorsque la montre 500 est portée.

Le transducteur ultrasonore 180 est notamment placé au milieu du fond de boîtier 610, de sorte à améliorer le contact entre le transducteur ultrasonore 180 et le poignet de l'utilisateur et minimiser les perturbations dues aux mouvements du poignet.

En variante, le transducteur ultrasonore 180 est arrangé dans le bracelet 502.

Le transducteur ultrasonore 180 est notamment configuré pour détecter l'artère radiale, qui est l'artère principale de l'avant-bras.

### TENSIOMÈTRE

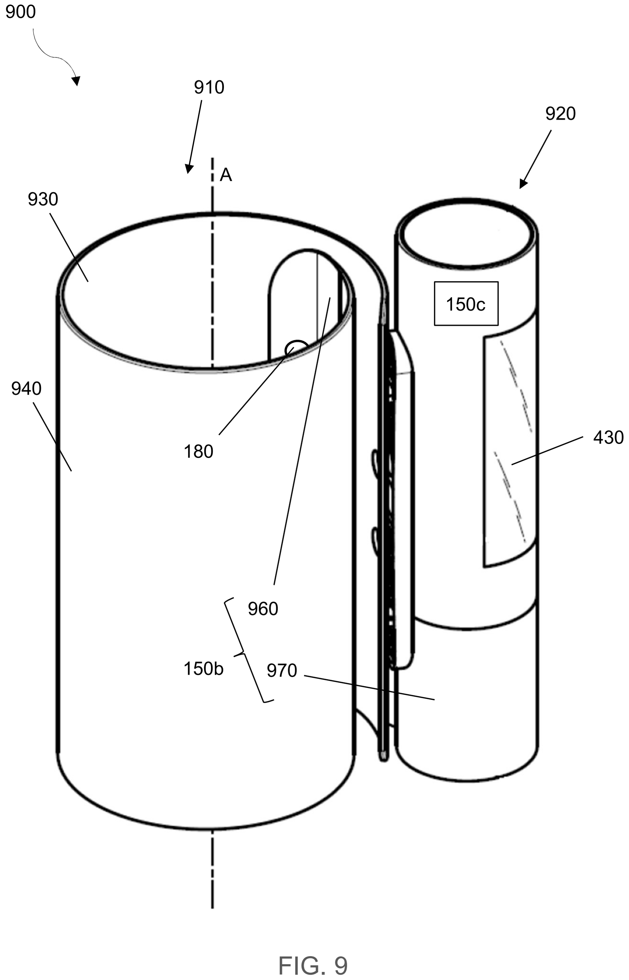

Dans un mode de réalisation, le dispositif portable 100 est configuré pour être placé au bras d'un utilisateur. Dans ce mode de réalisation, le dispositif est par exemple un tensiomètre 900, comme illustré sur la figure 9.

Le tensiomètre 900 comprend un brassard 910 et un boîtier de commande 920.

Dans le mode de réalisation illustré, le brassard 910 et le boîtier de commande 920 sont solidaires mécaniquement. En variante, le boîtier de commande 920 peut être uniquement relié au boîtier de commande 920 par un conduit flexible.

Le brassard 910 est configuré pour être enroulé autour d'un bras de l'utilisateur, en particulier autour de la partie du membre supérieur comprise entre l'épaule et le coude. En variante, le tensiomètre 900 peut être utilisé ailleurs, au niveau de l'avant-bras par exemple, ou au niveau du poignet. D'une manière générale, le brassard est configuré, en utilisation, pour entourer un membre supérieur de l'utilisateur s'étendant selon un axe principal A.

Le brassard 910 comprend une poche gonflable, non visible sur les figures, disposée entre une paroi intérieure 930 configurée pour être un contact du bras de l'utilisateur et une paroi extérieure 940.

Le brassard 910 est configuré pour passer d'une configuration développée à une configuration enroulée, comme illustrée sur la figure 4. Le brassard 910 peut comprendre un ressort, disposé entre la paroi intérieure 930 et la paroi extérieure 940, contraignant le brassard 910 dans la configuration enroulée.

En configuration développée, la longueur du brassard 910 selon la direction longitudinale, orthogonale à l'axe principal A, peut être comprise entre 20 cm et 40 cm. La hauteur le long de l'axe principal A peut être comprise entre 10 cm et 20 cm.

Le boîtier de commande 920 constitue l'enveloppe 110. Le boîtier de commande 920 peut s'étendre sensiblement selon l'axe principal A.

Le boîtier de commande 920 comprend une unité pneumatique comprenant notamment une pompe entraînée par un moteur électrique. La pompe est configurée pour gonfler la poche gonflable du brassard 910.

Le boîtier de commande 920 est configuré pour commander l'unité pneumatique et pour déterminer au moins la pression artérielle de l'utilisateur.

Le boîtier de commande 920 peut comprend l'affichage 430 configuré pour afficher notamment un menu de sélection des fonctions disponibles, le résultat des mesures effectuées et/ou les messages destinés à l'utilisateur, notamment les messages d'alerte.

Le boîtier de commande 920 présente généralement une forme cylindrique. Le diamètre du boîtier commande est par exemple inférieur à 40 mm, ce qui permet de loger tous les composants nécessaires à la mesure de manière compacte.

Le tensiomètre 900 comprend en outre une batterie 120, logée par exemple dans le boîtier de commande 920 et configurée pour fournir de l'énergie au tensiomètre 900, et en particulier aux composants électroniques du boîtier de commande 920.

### Capteur ECG

Dans un mode de réalisation, le capteur physiologique 150 est un capteur ECG 150b.

À cette fin, au moins deux électrodes, notamment trois électrodes de contact sont prévues, toutes trois intégrées dans le tensiomètre 900, sans qu'il soit nécessaire d'avoir des fils de liaison et des électrodes flottantes.

La première électrode 960 est disposée sur la paroi intérieure 930 du brassard 910 et présente une face orientée vers la peau de l'utilisateur.

La deuxième électrode 970 est disposée autour de la paroi externe du boîtier de commande 920, comme visible sur la figure 9. La deuxième électrode 970 comprend un matériau conducteur recouvrant au moins une partie du boîtier de commande 920.

Dans un mode de réalisation non représenté, une troisième électrode, peut être également disposée sur la paroi intérieure 930 du brassard 910 et présente également une face orientée vers la peau de l'utilisateur.

### Capteur optique

En variante ou en complément, dans un mode de réalisation non représenté, le capteur physiologique 150 est un capteur optique 150a. Le capteur optique 150a est disposé sur la paroi intérieure 930 du brassard 910 et est orientée vers la peau de l'utilisateur.

### Capteur oscillométrique

En variante ou en complément, le capteur physiologique 150 peut être un capteur oscillométrique 150c. Le capteur oscillométrique 150c est reliée à l'unité pneumatique pour mesurer une tension artérielle de l'utilisateur.

### Transducteur ultrasonore

Le transducteur ultrasonore 180 peut comprendre au moins un capteur ultrasonore miniaturisé. Par miniaturisé, on entend que la dimension transversale maximale du capteur ultrasonore est inférieure à 5 mm, notamment inférieur à 2 mm. Le capteur ultrasonore est notamment un émetteur-récepteur piézoélectrique.

Le transducteur ultrasonore 180 est disposé sur la paroi intérieure 930 du brassard 910 pour être en contact avec la peau de l'utilisateur lorsque le tensiomètre 900 est porté.

Le transducteur ultrasonore 180 est notamment configuré pour détecter l'artère brachiale, qui est l'artère principale du bras supérieur.

### BALANCE

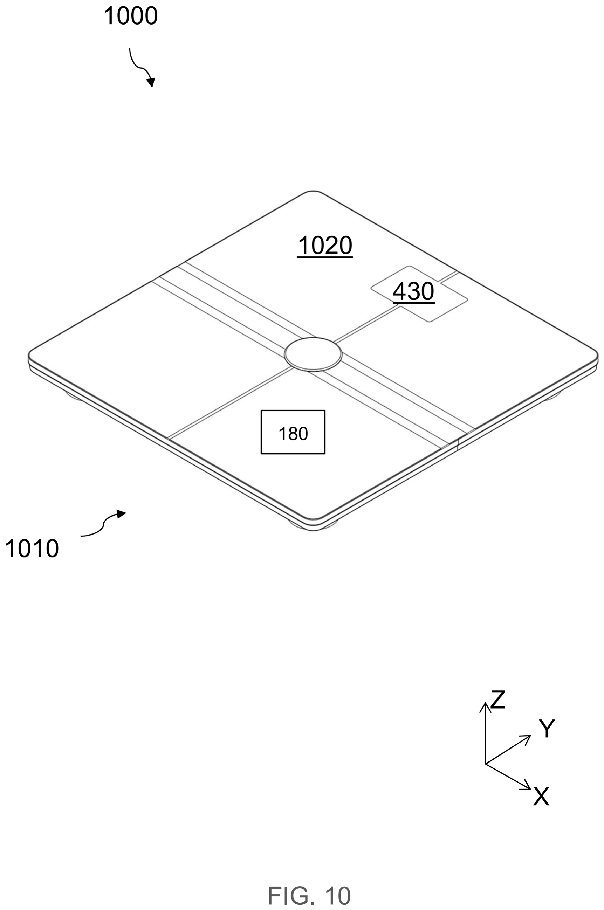

Dans un mode de réalisation, le dispositif 100 est configuré pour être au contact des pieds d'un utilisateur. Dans ce mode de réalisation, le dispositif est par exemple une balance, comme illustré sur la figure 10.

La balance 1000 se présente essentiellement sous la forme d'une base 1010 sur laquelle un utilisateur peut poser ses pieds, par exemple à plat. L'utilisateur peut être debout sur la base 1010 ou assis sur une chaise.

La balance 1000 peut comprendre en outre des capteurs de poids, comme des cellules de charge, apte à mesurer un poids de l'utilisateur. Les capteurs de poids peuvent permettent aussi d'effectuer un BCG (ballistocardiogramme), c'est-à-dire une mesure de variation du poids sous l'effet de l'éjection du sang du cœur.

La balance 1000 peur se présenter sous la forme d'un pèse-personne impédancemétrique configuré pour effectuer une mesure d'impédancemétrie (IPG).

La base 1010 peut comprendre un affichage 430, notamment un écran ou un affichage à LED ou à encre électronique, pour afficher des informations à destination de l'utilisateur.

La base 1010 comprend en outre une plaque de mesure 1020 apte à recevoir les pieds de l'utilisateur. La plaque de mesure 1020 transmet le poids de l'utilisateur vers les capteurs de poids.

La balance 1000 peut comprendre en outre une poignée reliée à la base 1010, non représentée, apte à être saisie par une moins une main de l'utilisateur.

Le document FR3131524 décrit par exemple une telle balance.

Le capteur physiologique 150 est disposé dans la base 1010 et éventuellement dans la poignée.

Le capteur physiologique 150 peut être un ballistographe ou ballistocardiographe, BCG, notamment constitué des capteurs de poids.

En variante ou en complément, le capteur physiologique 150 peut être capteur impédancemétrique, IPG.

En variante ou en complément, le capteur physiologique 150 peut être capteur ECG.

Le transducteur ultrasonore 180 est disposé dans la base 1010 pour être en contact avec la peau des pieds de l'utilisateur.

### AUTRES MODES DE REALISATION

Dans un mode de réalisation, le dispositif portable 100 est configuré pour être placé autour du cou de l'utilisateur. Le dispositif portable 100 se présente alors par exemple sous la forme d'un collier.

Dans un mode de réalisation, le dispositif portable 100 est configuré pour être placé au niveau de la tête de l'utilisateur. Le dispositif portable 100 se présente alors par exemple sous la forme d'écouteurs ou d'un bandeau autour de la tête.

Dans un mode de réalisation, le dispositif portable 100 est configuré pour être placé autour du torse de l'utilisateur. Le dispositif portable 100 se présente alors par exemple sous la forme d'une brassière ou d'une ceinture.

Dans un mode de réalisation, le dispositif portable 100 est configuré pour être placé autour de la jambe de l'utilisateur. Le dispositif portable 100 se présente alors par exemple sous la forme d'un bandeau autour de la cuisse.

L'homme du métier comprendra que le dispositif portable 100 peut être placé sur tout membre du corps de l'utilisateur permettant à la fois une mesure cardiaque et une mesure ultrasonore.

### METHODE D'ACTIVATION

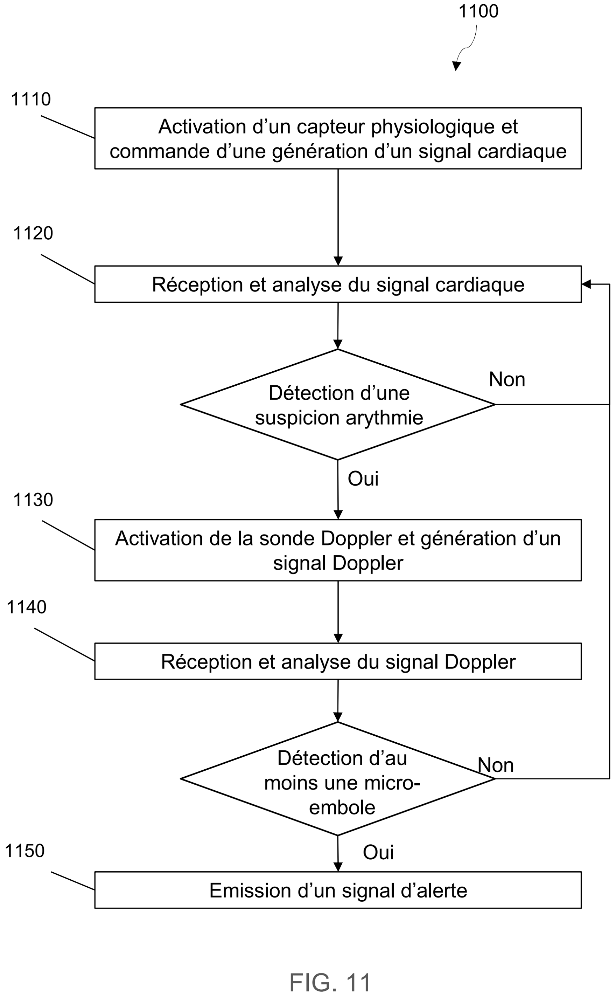

Une méthode d'activation 1100 du transducteur ultrasonore 180 mise en œuvre entièrement par l'unité de contrôle 140 va maintenant être décrite, en référence à la Figure 11. Comme mentionné précédemment, dans d'autres modes de réalisation, certaines étapes sont mises en œuvre par le terminal mobile 460 et/ou le serveur distant 470.

Le dispositif 100 est positionné sur un membre M d'un utilisateur, et notamment sur la peau P du membre M.

Initialement, le transducteur ultrasonore 180 est désactivé et n'effectue donc pas de mesures. Autrement dit, le transducteur ultrasonore 180 n'émet pas de signaux ultrasonores en direction du membre M.

Lors d'une étape 1110, le capteur physiologique 150 est activé par l'unité de contrôle 140. L'unité de contrôle 140 commande la génération d'un signal cardiaque par le capteur physiologique 150. La génération du signal cardiaque peut se faire en continu ou de manière périodique. Le capteur physiologique 150 est typiquement activé en continu.

Puis, lors d'une étape 1120, l'unité de contrôle 140 reçoit le signal cardiaque généré par le ou les capteur physiologique 150 et analyse le signal cardiaque. L'analyse se fait en continu, et particulièrement en temps réel, par exemple avec une fenêtre temporelle glissante, comme décrit précédemment.

En réponse à une détection d'une arythmie cardiaque dans l'analyse du signal cardiaque à l'étape 1120, l'unité de contrôle 140 active le transducteur ultrasonore 180 lors d'une étape 1130 et commande la génération d'un signal Doppler par le transducteur ultrasonore 180. L'activation 1130 se fait en réponse à la détection d'une arythmie, sans délai autre que les délais incompressibles liés à la technique.

Puis, lors d'une étape 1140, l'unité de contrôle 140 reçoit le signal Doppler généré et analyse le signal Doppler.

En réponse à une détection d'une micro-embole ME dans l'analyse du signal Doppler à l'étape 1140, l'unité de contrôle 140 génère un signal d'alerte dans une étape 1150, notamment à destination de l'utilisateur et/ou d'un personnel médical.

Puis, le transducteur ultrasonore 180 est désactivé par l'unité de contrôle 140, notamment pour économiser la batterie 120 du dispositif 100.

La méthode 1100 permet donc une détection non invasive et précise des micro-emboles ME chez des utilisateurs atteints de fibrillation auriculaire. En intégrant un transducteur ultrasonore 180 dans un dispositif portable 100, tel que par exemple une montre connectée 500 ou un tensiomètre 900, il est possible de détecter les micro-emboles ME à la suite de la détection d'une arythmie, et ainsi permettre une intervention médicale rapide, ce qui est crucial pour prévenir les dommages causés par les AVC.

En outre, l'activation du transducteur ultrasonore 180 uniquement en cas de détection d'arythmie permet une utilisation de la batterie 120 du dispositif portable 100 optimisée rendant possible un suivi long-terme et continu pour l'utilisateur.

## Revendications

1. Dispositif (100) configuré pour être positionné sur un membre (M) d'un utilisateur, le dispositif (100) comprenant :
• un capteur physiologique (150) configuré pour générer un signal cardiaque caractéristique d'une activité cardiaque de l'utilisateur,
• un transducteur ultrasonore (180) configuré pour générer un signal Doppler caractéristique d'une circulation sanguine dans le membre (M),
• une unité de contrôle (140) configurée pour :
∘ recevoir un signal cardiaque du capteur physiologique (150),
∘ analyser le signal cardiaque reçu, et
∘ en réponse à une détection d'une suspicion d'arythmie cardiaque dans l'analyse du signal cardiaque, activer le transducteur ultrasonore (180) pour générer un signal Doppler.

2. Dispositif (100) selon la revendication 1, dans lequel l'arythmie est une fibrillation auriculaire.

3. Dispositif (100) selon l'une quelconque des revendications précédentes, dans lequel le capteur physiologique (150) est un capteur optique (150a) configuré pour effectuer une mesure du signal cardiaque en continu et l'unité de contrôle (140) est configurée pour analyser le signal cardiaque en continu.

4. Dispositif (100) selon l'une quelconque des revendications précédentes, dans lequel le capteur physiologique (150) est un capteur ECG (150b), un capteur oscillométrique (150c), un ballistocardiographe, BCG, et/ou un capteur impédancemétrique, IPG.

5. Dispositif (100) selon l'une quelconque des revendications précédentes, dans lequel le dispositif (100) est un dispositif portable.

6. Dispositif (100) selon l'une quelconque des revendications précédentes, dans lequel le signal Doppler est généré concomitamment au signal cardiaque ou moins de 10 secondes, voire 5 secondes après le signal cardiaque.

7. Dispositif (100) selon l'une quelconque des revendications précédentes, dans lequel l'unité de contrôle (140) est configurée pour recevoir le signal Doppler et configurée pour détecter au moins une micro-embole (ME) dans l'artère par analyse du signal Doppler.

8. Dispositif (100) selon la revendication 7, dans lequel l'unité de contrôle (140) est configurée pour émettre un signal d'alerte en cas de détection d'un débit de micro-emboles (ME) supérieur à un seuil d'alerte prédéterminé, par exemple un message d'alerte à l'utilisateur et/ou à une personne tierce.

9. Dispositif (100) selon l'une quelconque des revendications précédentes, dans lequel le dispositif (100) est une montre (500).

10. Dispositif (100) selon l'une quelconque des revendications précédentes, dans lequel le dispositif (100) est un tensiomètre (900).
